# DEMANDE DE BREVET EUROPEEN

(11) **EP 3 760 740 A1**
(43) Date de publication de la demande: **06.01.2021**
(21) Numéro de dépôt: 19305920.1
(22) Date de dépôt: 05.07.2019
(51) Int. Cl.: C12Q 1/6883

(54) **UTILISATION DU NIVEAU D'ADN LIBRE COMME MARQUEUR DE L'ENDOMETRIOSE**

(71) Demandeur: Hazout, André, 75016 Paris (FR); Advanced Technologies Laboratory, 78320 La Verriere (FR)
(72) Inventeur: HAZOUT, André, 75016 Paris (FR); BEN KHALIFA, Moncef, 78320 Le Mesnil-Saint-Danis (FR)
(74) Mandataire: Santarelli

(57) **Abrégé**

L'invention porte sur une méthode de diagnostic non invasif de l'endométriose, basé sur la mesure du taux d'ADN acellulaire dans un échantillon biologique d'un individu, dans laquelle un taux d'ADN acellulaire supérieur à seuil de référence indique que l'individu est susceptible d'avoir de l'endométriose, et un taux d'ADN acellulaire mesuré inférieur à seuil de référence permet d'écarter le diagnostic d'endométriose évolutive. La méthode peut également comprendre une étape de mesure du niveau de méthylation d'au moins 15 gènes impliqués dans l'endométriose, permettant de caractériser le potentiel évolutif de l'endométriose chez la patiente testée.

## Description

### DOMAINE DE L'INVENTION

La présente invention concerne le domaine du diagnostic non invasif de l'endométriose. Plus particulièrement, l'invention propose des marqueurs sériques (niveau d'ADN acellulaire et marqueurs épigénétiques) permettant de poser un diagnostic de l'endométriose et d'en caractériser le potentiel évolutif.

### ARRIERE-PLAN TECHNOLOGIQUE

L'endométriose est une maladie inflammatoire chronique, oestrogéno-dépendante, due à la migration des cellules de l'endomètre en-dehors de la cavité utérine, principalement au niveau des organes et des tissus du pelvis. Cet état inflammatoire associé à des douleurs pelviennes et, parfois, à un état de stérilité, atteint 3 à 10% des femmes jeunes en âge de procréer.

La maladie est hétérogène, allant de lésions superficielles péritonéales et séreuses à des kystes d'endométriose dans les ovaires (endométriome), des nodules dans l'endométriose profonde, et peut souvent être accompagnée de fibrose et d'adhérences. L'endométriose peut survenir chez une jeune fille prépubère algique (Marsh et Laufer, 2005) puis de la puberté à la ménopause, où la cyclicité menstruelle et la douleur disparaissent.

Nous savons que la croissance des cellules endométriales ectopiques est dépendante des oestrogènes. L'endométriose est largement perçue comme une régurgitation du sang des règles avec une migration des cellules de l'endomètre vers tous les organes environnants et parfois au-delà : abdomen, poumons, cerveau et ailleurs.

Très rarement, une endométriose a également été constatée dans le tractus génito-urinaire de l'homme (Beckman *et al.,* 1985 ; Fukunaga, 2012 ; Rei et Feloney, 2018). Vingt-deux cas d'endométrioses hépatiques ont aussi été publiés (Liu *et al.,* 2015). Ceci est un argument contre la théorie prédominante du flux rétrograde telle qu'elle a été étudiée dans l'endométriose féminine. Il est essentiel d'expliquer pourquoi seulement 10% des femmes développent une endométriose alors que la régurgitation des règles survient dans 76% à 90% des femmes en âge de procréer (Blumenkrantz *et al.,* 1981 ; Halme *et al.,* 1984).

Le diagnostic d'endométriose est posé lors d'une laparoscopie et confirmé par l'analyse des lésions extraites lors d'une laparoscopie opératoire. Le traitement est souvent médical pour les formes légères à modérées (stades I et II) et chirurgical immédiatement ou après l'hormonothérapie pour les formes sévères (stades III et IV).

L'endométriose a été décrite alternativement comme une maladie hormonale ou immunitaire, et une maladie génétique déclenchée par l'exposition à des facteurs environnementaux. En outre, de nombreuses études suggèrent diverses aberrations épigénétiques dans la pathogenèse de l'endométriose.

L'hétérogénéité du phénotype de la maladie est authentifiée par un grand nombre de laparoscopies faussement négatives chez la femme symptomatique. Aussi les examens anatomopathologiques, immuno-histochimiques et épigénétiques des lésions ne se sont pas révélées fiables (Soo Hyun Ahn *et al.,* 2017), notamment concernant la méthylation de l'ADN génomique du récepteur B de la progestérone, de l'e-cadherine, de l'homeobox A10 (HOXA10), du récepteur d'oestrogène b, du facteur stéroïdogène 1(SF1) et de l'aromatase.

L'expression aberrante de l'ADN méthyltransférase, qui adjoint un groupe méthyle en position 5 des bases cytosines dans l'îlot CpG (Cytosine phosphate Guanine) du promoteur du gène, rendant silencieuse l'expression génique correspondante, a été démontrée dans l'endométriose (Nasu *et al.,* 2011).

Dans le sang humain, la présence d'ADN libre circulant sans cellules (cfDNA) a été rapportée en 1948 par Mendel et Metais (Mandel et Metais, 1948). Le cfDNA a été étudié dans un large éventail de conditions physiologiques et pathologiques, notamment les troubles inflammatoires, le stress oxydatif, la stérilité du couple (EP2879696B1) et les tumeurs malignes.

Chez les individus sains, lors de la phagocytose, les corps apoptotiques ou nécrotiques sont ingérés par les macrophages. Ainsi, le phénomène de relargage de l'ADN libre ne peut pas avoir lieu. En revanche, lorsque les fragments d'ADN subsistent dans les nucléosomes qui sont relargués par les macrophages, ils sont protégés de la dégradation enzymatique et ainsi restent dans la circulation sanguine.

Le cfDNA est composé d'acides nucléiques double brins au poids moléculaire plus faible que l'ADN génomique. La taille de ces fragments génomiques est variable, allant pour les plus courts de 70 à 200 pb et pour les plus longs jusqu'à 21 kb. Le cfDNA est présent chez le sujet sain à des concentrations sanguines évaluées entre 50 et 250 ng/ml (EP2879696B1).

Les mécanismes biologiques de libération de l'ADN libre dans le sang ne sont pas entièrement connus. Des fragments d'ADN libre peuvent provenir de cellules nécrotiques englouties par les macrophages qui se libèrent alors partiellement. Selon cette hypothèse, les niveaux de cfDNA devraient être corrélés à l'étendue de la nécrose et/ou l'apoptose cellulaire.

La clairance du cfDNA de la circulation sanguine est rapide (demi-vie : 16,3 min.) ; il est connu que le cfDNA est sensible aux nucléases plasmatiques, mais les clairances rénale et hépatique participent également à son élimination.

Le cfDNA peut être isolé du plasma et du sérum, mais le sérum contient une concentration d'ADN environ 6 fois supérieure (incorporation dans les cellules).

Les niveaux d'ADN et les schémas de fragmentation offrent des possibilités intéressantes à des fins diagnostiques et pronostiques.

À ce jour, seule une étude de Zachariah (2009) a montré un cfDNA nucléaire et mitochondrial significativement plus élevé dans un groupe de femmes atteintes d'endométriose minime à légère que dans un groupe témoin (p = 0,046). Le seuil à partir d'une courbe ROC montrait une sensibilité à 70% et une spécificité à 87%. L'auteur concluait que le cfDNA circulant pouvait constituer un biomarqueur potentiel de l'endométriose minime et légère.

### RESUME DE L'INVENTION

La présente invention porte sur une méthode de diagnostic non invasif (*in vitro*) de l'endométriose, basé sur la mesure du taux d'ADN acellulaire dans un échantillon biologique d'un individu, dans laquelle un taux d'ADN acellulaire supérieur à seuil de référence indique que l'individu est susceptible d'avoir de l'endométriose, et un taux d'ADN acellulaire mesuré inférieur à seuil de référence (identique ou différent du premier) permet d'écarter le diagnostic d'endométriose évolutive.

Selon un autre aspect, l'invention porte sur une méthode qui permet non seulement d'établir un diagnostic d'endométriose, mais également de déterminer le potentiel évolutif de cette endométriose, par la mesure du niveau de méthylation d'au moins 15 gènes impliqués dans l'endométriose. Selon cet aspect de l'invention, (i) une hyperméthylation des gènes sélectionnés parmi CALD1, RRP1, FN1, FAM87B, TCEAL6, RPL29P2, ATP11A-AS1, DIP2C, SLCO2B1, RMI2, MIR3170, LINC01007, TSPAN17, MIR4693, HYOU1, TLR4, ADGRL3, IL6, VIRMA, MKRN1, INSIG1, ROR2, MRPL3, FMNL2, TMEM19, ZNF438, LINC01192, RCBTB1, TSPAN33, NKD2, FGFR2, TPRG1, MIR4644, FOXO4, FSTL1 et CLMN, et/ou (ii) une hypométhylation des gènes sélectionnés parmi NT5C2, NAV1, SOD3, C3, UBE3A, MIR4655, MYO5C, COX6C, MIR6133, BRSK2, MIR4277, MIR4251, MN1, MIR3666, AZIN1, MIR4251, SLC37A2, FZD10, STAU2-AS1, TDRD5, USP1, ACVR2A, FBXO38, FASN, MKRN9P et PCCA-AS1, constituent des signes évocateurs d'une endométriose dont le potentiel évolutif est d'autant plus important que l'hyper- ou hypo-méthylation mesurée est importante.

La présente invention concerne également des kits pour mettre en oeuvre les méthodes de l'invention.

### LEGENDES DES FIGURES

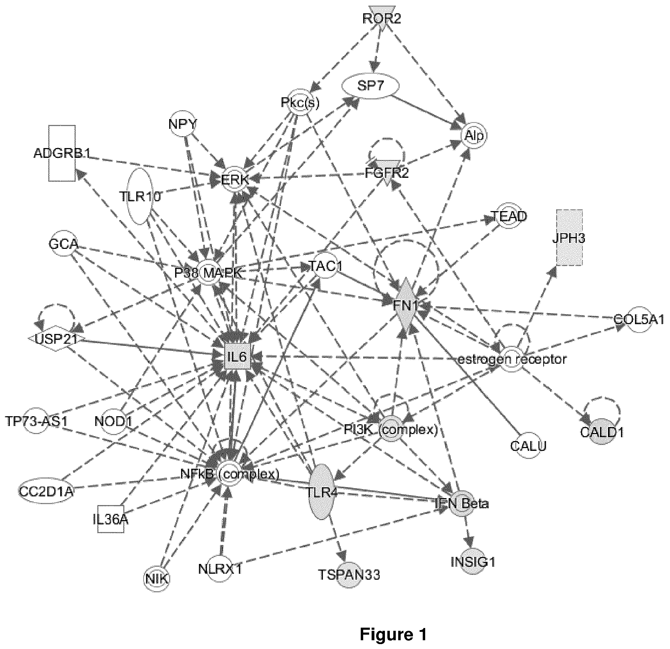

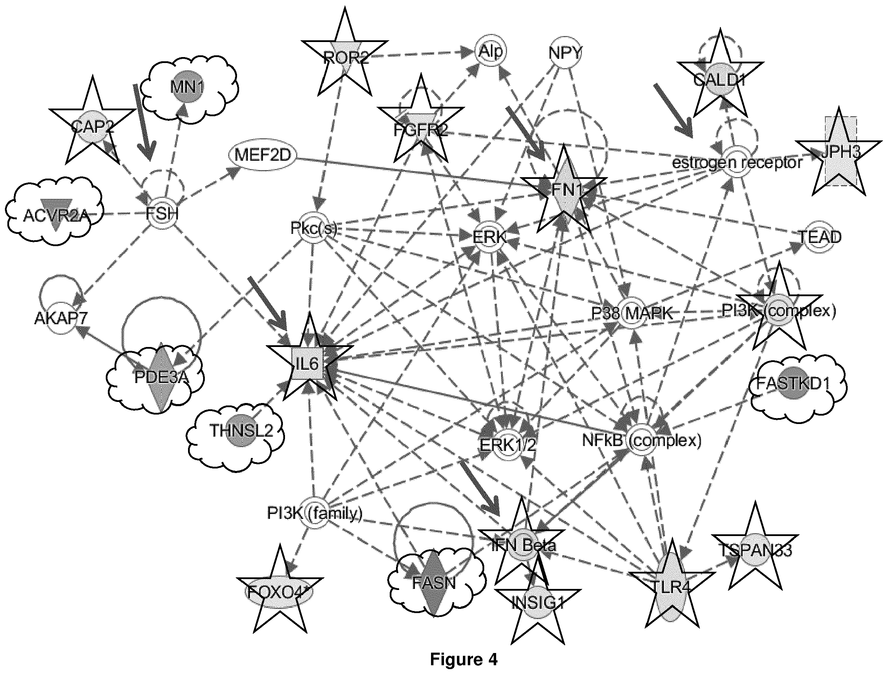

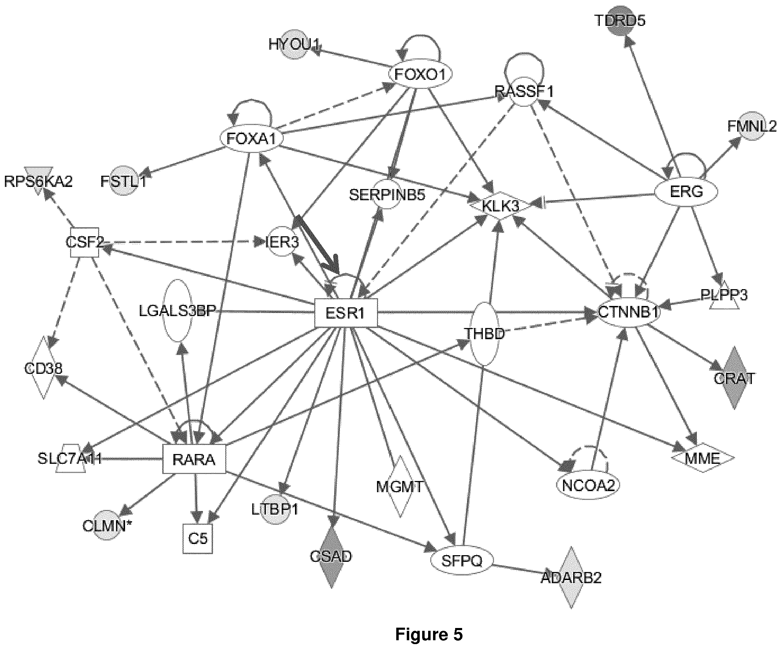

**Figure 1** : **réseau de gènes générés Ingenuity Pathway Analysis (IPA), obtenu uniquement à partir des gènes hyperméthylés chez les femmes endométriosiques.** Les lignes continues entre les noeuds indiquent une interaction moléculaire directe entre les gènes connectés, tandis que les lignes en pointillés indiquent une interaction fonctionnelle indirecte entre les gènes. Figures 4 et 5 en considérant tous les gènes différentiellement méthylés (DMG : hyper- et hyométhylés).

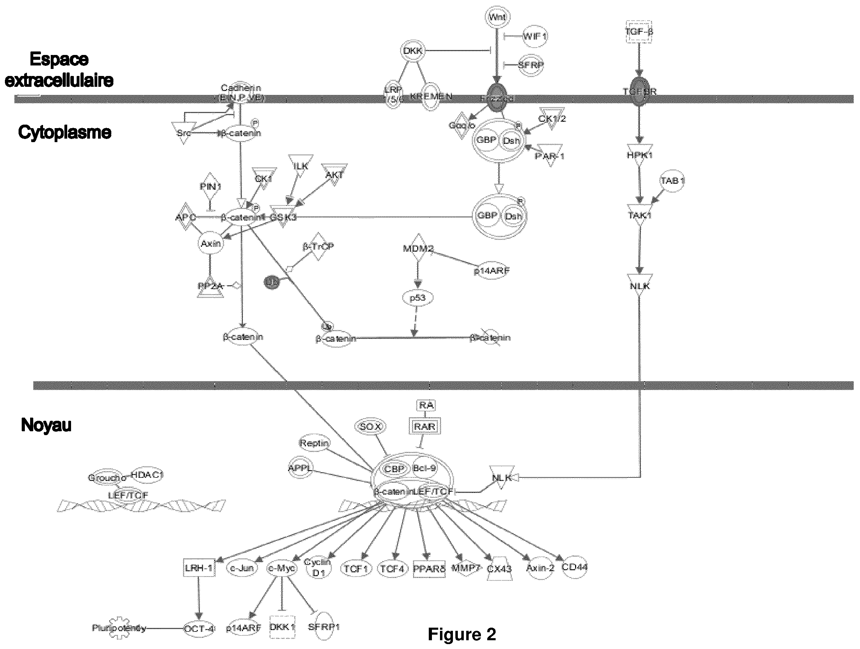

**Figure 2** **: Voies de conduction transmembranaire : récepteur du TGF Béta et protéines FRIZZLED.**

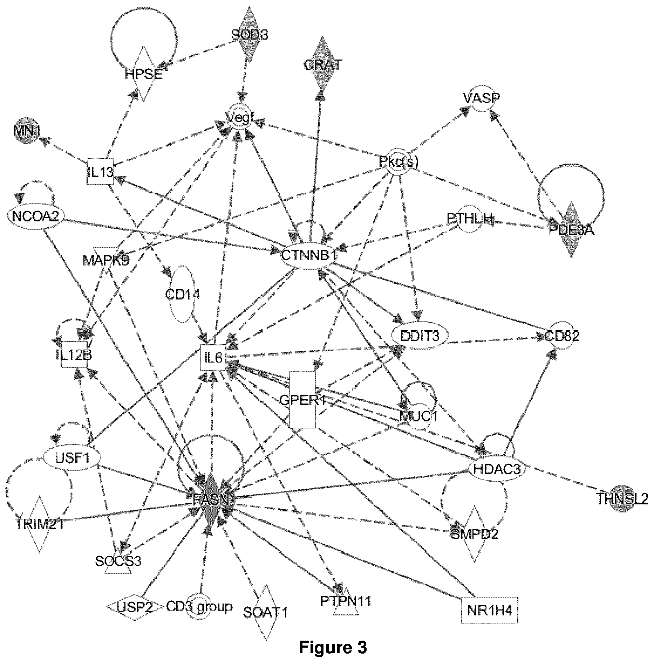

**Figure 3** **: réseau de gènes générés Ingenuity Pathway Analysis (IPA), obtenu uniquement à partir des gènes hypométhylés chez les femmes endométriosiques.** Les lignes continues entre les noeuds indiquent une interaction moléculaire directe entre les gènes connectés, tandis que les lignes en pointillés indiquent une interaction fonctionnelle indirecte entre les gènes.
**Figure 4** **: réseau de gènes générés Ingenuity Pathway Analysis (IPA), obtenu en considérant tous les gènes différentiellement méthylés (DMG : hyper- et hyométhylés).** Les lignes continues entre les noeuds indiquent une interaction moléculaire directe entre les gènes connectés, tandis que les lignes en pointillés indiquent une interaction fonctionnelle indirecte entre les gènes.
**Figure 5** **: réseau de gènes générés Ingenuity Pathway Analysis (IPA), obtenu en considérant tous DMG (hyper- et hyométhylés).** Les lignes continues entre les noeuds indiquent une interaction moléculaire directe entre les gènes connectés, tandis que les lignes en pointillés indiquent une interaction fonctionnelle indirecte entre les gènes.

### DESCRIPTION DETAILLEE

Selon un premier aspect, la présente invention porte sur une méthode de diagnostic *in vitro* de l'endométriose, comprenant
(i) la mesure du taux d'ADN acellulaire dans un échantillon biologique d'un individu et
(ii) la comparaison du taux d'ADN acellulaire avec un seuil prédéterminé,
dans laquelle un taux d'ADN acellulaire supérieur au seuil prédéterminé indique que l'individu est susceptible d'avoir de l'endométriose, et un taux d'ADN acellulaire mesuré inférieur au seuil prédéterminé permet d'écarter une endométriose évolutive.

Cette méthode peut être mise en oeuvre pour établir, de manière non invasive, un diagnostic d'endométriose chez un humain, en particulier chez une femme, notamment chez une femme en âge de procréer.

Selon une mise en oeuvre particulière de la méthode ci-dessus, l'échantillon à partir duquel on mesure le taux d'ADN acellulaire est un échantillon de sérum.

La quantification du cfDNA peut être réalisée comme indiqué dans la partie expérimentale ci-dessous, ou à l'aide d'autres méthodes décrites dans la littérature scientifique, notamment des méthodes fluorométriques ou spectrophotométriques telles que QUBIT® (Life Technologies) ou NANODROP™ (Thermo Scientific). Récemment, l'analyse de l'ADN acellulaire a été abondamment décrite dans des méthodes de diagnostic de certains cancers ou dans le diagnostic prénatal d'anomalies chromosomiques. Aussi, plusieurs technologies pour isoler et analyser l'ADN acellulaire ont été décrites, tant dans les publications scientifiques que la littérature brevets. L'homme du métier peut parfaitement choisir, parmi les multiples technologies décrites, celle qui lui semble appropriée pour mettre en oeuvre l'invention.

Selon une autre mise en oeuvre particulière de l'invention, la méthode comprend également une étape (iii) de détermination du niveau de méthylation de certains gènes impliqués dans l'endométriose, toujours à partir de l'ADN acellulaire présent dans un échantillon de sérum de l'individu. Dans une réalisation préférée de l'invention, cette étape comprend l'analyse de la méthylation d'au moins 15 gènes sélectionnés parmi les gènes décrits dans le tableau ci-dessous :

**Tableau 1 : gènes présentant un profil de méthylation (hyper- ou hypo-méthylation) différent chez les patients endométriosiques.**

| **Nom** | **Identifiant GenBank** | **Nom** | **Identifiant GenBank** | **Nom** | **Identifiant GenBank** |
|---|---|---|---|---|---|
| CALD1 | NM_033140 | ROR2 | NM_004560 | MYO5C | NM_018728 |
| RRP1 | NM_003683 | MRPL3 | NM_007208 | COX6C | NM_004374 |
| FN1 | NM_212482 | FMNL2 | NM_052905 | MIR6133 | NR_106749 |
| FAM87B | NR_103536 | TMEM19 | NM_018279 | BRSK2 | NM_001256629 |
| TCEAL6 | NM_001006938 | ZNF438 | NM_182755 | MIR4277 | NR_036240 |
| RPL29P2 | NR_002778 | LINC01192 | NR_033945 | MIR4251 | NR_036215 |
| ATP11A-AS1 | NR_046661 | RCBTB1 | NM_018191 | MN1 | NM_002430 |
| DIP2C | NM_014974 | TSPAN33 | NM_178562 | MIR3666 | NR_037439 |
| SLCO2B1 | NM_007256 | NKD2 | NM_001271082 | AZIN1 | NM_148174 |
| RMI2 | NM_152308 | FGFR2 | NR_073009 | MIR4251 | NR_036215 |
| MIR3170 | NR_036129 | TPRG1 | NM_198485 | SLC37A2 | NM_198277 |
| LINC01007 | NR_103749 | MIR4644 | NR_039787 | FZD10 | NM_007197 |
| TSPAN17 | NM_012171 | FOXO4 | NM_005938 | STAU2-AS1 | NR_038406 |
| MIR4693 | NR_039842 | FSTL1 | NM_007085 | TDRD5 | NM_001199091 |
| HYOU1 | NM_006389 | CLMN | NM_024734 | USP1 | NM_003368 |
| TLR4 | NM_138554 | NT5C2 | NM_012229 | ACVR2A | NM_001616 |
| ADGRL3 | NM_015236 | NAV1 | NM_020443 | FBXO38 | NM_001271723 |
| IL6 | NM_000600 | SOD3 | NM_003102 | FASN | NM_004104 |
| VIRMA | NM_015496 | C3 | NM_000064 | MKRN9P | NR_033410 |
| MKRN1 | NR_117084 | UBE3A | NM_130839 | PCCA-AS1 | NR_047686 |
| INSIG1 | NM_198337 | MIR4655 | NR_039799 | | |

Lors de cette analyse, on considère qu'une hyperméthylation ou une hypométhylation de ces gènes, par rapport au niveau de méthylation des mêmes gènes dans une population d'individus non endométriosiques, constitue un signe évocateur d'une endométriose. Plus précisément, sont considérés comme signes évocateurs d'une endométriose les profils de méthylations ci-dessous :
(i) une hyperméthylation des gènes sélectionnés parmi CALD1, RRP1, FN1, FAM87B, TCEAL6, RPL29P2, ATP11A-AS1, DIP2C, SLCO2B1, RMI2, MIR3170, LINC01007, TSPAN17, MIR4693, HYOU1, TLR4, ADGRL3, IL6, VIRMA, MKRN1, INSIG1, ROR2, MRPL3, FMNL2, TMEM19, ZNF438, LINC01192, RCBTB1, TSPAN33, NKD2, FGFR2, TPRG1, MIR4644, FOXO4, FSTL1 et CLMN et/ou
(ii) une hypométhylation des gènes sélectionnés parmi NT5C2, NAV1, SOD3, C3, UBE3A, MIR4655, MYO5C, COX6C, MIR6133, BRSK2, MIR4277, MIR4251, MN1, MIR3666, AZIN1, MIR4251, SLC37A2, FZD10, STAU2-AS1, TDRD5, USP1, ACVR2A, FBXO38, FASN, MKRN9P et PCCA-AS1.

Par « hyperméthylation d'un gène » (ou « hypométhylation d'un gène »), on entend ici un niveau de méthylation supérieur (ou inférieur) au niveau taux de normométhylation de 15% de la séquence codante du gène, entraînant une augmentation (ou une répression) de l'expression de ce gène.

Parmi les 36 gènes hyperméthylés (liste (i) ci-dessus) et les 26 gènes hypométhylés (liste (ii) ci-dessus) chez les patientes endométriosiques, les inventeurs en ont identifiés 15 dont l'analyse permet, à elle seule, de différencier, dans la cohorte étudiée, les patientes endométriosiques des autres. Cette liste est constituée des gènes suivants: CALD1, RRP1, FN1, FAM87B, TCEAL6, RPL29P2, ATP11A-AS1, DIP2C, SLCO2B1, RMI2, FBX038, ACVR2A, USP1, TDRD5 et STAU2-AS1. Bien entendu, une analyse similaire sur une cohorte plus importante est susceptible d'entraîner des ajouts ou remplacements dans cette liste, sans pour autant sortir de la portée de la présente demande.

Selon une mise en oeuvre particulière de l'invention, on mesure le niveau de méthylation d'au moins 5 gènes choisis dans le groupe constitué par CALD1, RRP1, FN1, FAM87B, TCEAL6, RPL29P2, ATP11A-AS1, DIP2C, SLCO2B1, RMI2, FBX038, ACVR2A, USP1, TDRD5 et STAU2-AS1.

Par exemple, la méthode peut être mise en oeuvre en mesurant le niveau de méthylation de 5, 6, 7, 8, 9, 10, 11, 12, 13, 14 ou 15 gènes choisis dans le groupe constitué par CALD1, RRP1, FN1, FAM87B, TCEAL6, RPL29P2, ATP11A-AS1, DIP2C, SLCO2B1, RMI2, FBX038, ACVR2A, USP1, TDRD5 et STAU2-AS1.

Lors de la mise en oeuvre de cet aspect de l'invention, le profil de méthylation de l'ADN peut être mesuré par n'importe quel procédé décrit dans la littérature scientifique.

En particulier, trois grandes méthodes moléculaires, fondées sur une détection enzymatique, immunologique ou chimique, permettent de cartographier les cytosines méthylées. Dans le cadre de la présente invention, ces différentes techniques peuvent être combinées à des méthodes d'hybridation sur puces ou de séquençage à haut débit pour une résolution plus détaillée. Les quatre techniques les plus couramment utilisées sont MeDIP-seq, WGBS, RRBS et 450K Bead Array. Ces différentes méthodes peuvent être aisément mises en oeuvre par l'homme du métier, grâce à la disponibilité de protocoles détaillés dans la littérature, de kits commerciaux et de laboratoires spécialisés. Ces différentes méthodes produisent des résultats concordants, avec cependant des sensibilités variables de détection de régions différentiellement méthylées entre échantillons. Aussi, dans le cadre de la présente invention, le niveau « normal » de méthylation pour les gènes analysés doit être calibré en utilisant la technique qui sera utilisée pour mesurer le niveau de méthylation des gènes à partir de l'échantillon biologique.

### Méthode de détection immunologique

Les anticorps spécifiques des cytosines méthylées permettent une détection par immunoprécipitation, selon une méthode appelée MeDIP (*Methylated DNA ImmunoPrecipitation*). Classiquement, l'ADN est fragmenté par sonication, et les fragments les plus méthylés seront les plus favorablement précipités en présence de l'anticorps, permettant d'obtenir une fraction du génome enrichie en méthylation. Dans le cadre de la présente invention, l'étape de sonication n'est pas indispensable, étant donné le caractère fragmenté de l'ADN acellulaire. Couplée au séquençage à haut débit (MeDIP-seq), cette méthode permet de mesurer une densité locale de méthylation, avec une résolution d'environ 200 nucléotides correspondant à la taille moyenne des fragments, avec un coût raisonnable. Elle autorise une couverture complète du génome, avec cependant un biais pour les régions les plus riches en motifs CpG.

### Méthodes de détection chimique

Le seul outil permettant d'interroger le statut de méthylation à l'échelle de la cytosine individuelle est fondé sur le bisulfite. En présence de ce composé chimique, les cytosines sont converties en uracile, alors que les cytosines méthylées ne sont pas affectées. Cette méthode permet ainsi une lecture de la méthylation par analyse des polymorphismes nucléotidiques simples (SNP), dans lesquels un T correspond à une cytosine non modifiée et un C à une cytosine méthylée sur le génome de référence avant conversion.

Le séquençage complet de l'ADN après traitement au bisulfite, ou WGBS (*Whole-Genome Bisulfite Sequencing*), permet d'accéder au statut de méthylation de toutes les cytosines, représentant l'excellence de toutes les méthodes de cartographie de la méthylation génomique.

Le RRBS (*Reduced Representation Bisulfite Sequencing*) est une technique dérivée du WGBS, fondée sur la sélection préalable des régions génomiques riches en CpG par l'utilisation d'enzymes de restriction. En réduisant le nombre de fragments à séquencer, le coût et la profondeur du séquençage s'en trouvent grandement améliorés, dans le même ordre de grandeur que le MeDIP-seq.

Enfin, l'ADN converti au bisulfite peut aussi être hybridé sur une puce d'oligonucléotides, comprenant des oligonucléotides spécifiques des gènes différentiellement méthylés chez les patients endométriosiques.

Lors de la mise en oeuvre de la méthode ci-dessus, le niveau de méthylation des gènes mesurés permet de confirmer, mais également de préciser le diagnostic d'endométriose.

En particulier, selon une mise en oeuvre particulière de l'invention, le niveau de méthylation des gènes mesurés permet de caractériser le potentiel évolutif de l'endométriose. En effet, ce potentiel évolutif est d'autant plus important que le profil de méthylation des gènes analysés est caractéristique des patientes endométriosiques. En particulier, la potentiel évolutif sera considéré comme particulièrement important si on mesure une hyperméthylation significative ou importante d'au moins 7 gènes sélectionnés parmi CALD1, RRP1, FN1, FAM87B, TCEAL6, RPL29P2, ATP11A-AS1, DIP2C, SLCO2B1, RMI2, MIR3170, LINC01007, TSPAN17, MIR4693, HYOU1, TLR4, ADGRL3, IL6, VIRMA, MKRN1, INSIG1, ROR2, MRPL3, FMNL2, TMEM19, ZNF438, LINC01192, RCBTB1, TSPAN33, NKD2, FGFR2, TPRG1, MIR4644, FOXO4, FSTL1 et CLMN, et/ou une hypométhylation significative ou importante d'au moins 3 gènes sélectionnés parmi NT5C2, NAV1, SOD3, C3, UBE3A, MIR4655, MYO5C, COX6C, MIR6133, BRSK2, MIR4277, MIR4251, MN1, MIR3666, AZIN1, MIR4251, SLC37A2, FZD10, STAU2-AS1, TDRD5, USP1, ACVR2A, FBXO38, FASN, MKRN9P et PCCA-AS1. L'homme de métier est à même de définir les valeurs à partir desquelles il doit considérer une hyper- ou hypométhylation comme significative ou importante. A titre de valeur indicative, on peut considérer l'hyper- ou hypométhylation comme significative si la valeur absolue du différentiel de méthylation est supérieure à 10, et comme importante si la valeur absolue du différentiel de méthylation est supérieure à 20.

Ainsi, selon une mise en oeuvre particulière de l'invention, une hyperméthylation importante des gènes CALD1, RRP1, FN1, FAM87B, TCEAL6, RPL29P2, ATP11A-AS1, DIP2C, SLCO2B1 et RMI2 et une hypométhylation importante des gènes FBX038, ACVR2A, USP1, TDRD5 et STAU2-AS1 conduisent au diagnostic d'une endométriose susceptible de s'aggraver rapidement.

La présente invention porte également sur une trousse ou kit de diagnostic pour déterminer le potentiel évolutif d'une endométriose, comprenant des réactifs pour mesurer le niveau de méthylation d'au moins 15 gènes sélectionnés parmi ceux cités dans le tableau 1 ci-dessus.

Selon un mode de réalisation particulier, le kit selon l'invention comprend des amorces et/ou des sondes spécifiques d'au moins 15 gènes tels que définis ci-dessus. En particulier, le kit peut comprendre des amorces et/ou des sondes spécifiques de 15 gènes cités dans le Tableau 1, parmi lesquels 5, 6, 7, 8, 9, 10, 11, 12, 13, 14 ou 15 gènes sont choisis dans le groupe constitué par CALD1, RRP1, FN1, FAM87B, TCEAL6, RPL29P2, ATP11A-AS1, DIP2C, SLCO2B1, RMI2, FBX038, ACVR2A, USP1, TDRD5 et STAU2-AS1.

Selon un autre mode de réalisation particulier, le kit selon l'invention comprend une puce oligonucléotidique sensible à la méthylation comportant des olignocléotides spécifiques d'au moins 15 gènes tels que définis ci-dessus. En particulier, la puce peut comprendre des oligonucléotides spécifiques de 15 gènes cités dans le Tableau 1, parmi lesquels 5, 6, 7, 8, 9, 10, 11, 12, 13, 14 ou 15 gènes sont choisis dans le groupe constitué par CALD1, RRP1, FN1, FAM87B, TCEAL6, RPL29P2, ATP11A-AS1, DIP2C, SLCO2B1, RMI2, FBX038, ACVR2A, USP1, TDRD5 et STAU2-AS1.

Selon un autre mode de réalisation particulier, le kit selon l'invention comprend également des anticorps spécifiques des cytosines méthylées.

Selon un autre mode de réalisation particulier, le kit selon l'invention comprend également des réactifs pour mesurer le niveau d'ADN acellulaire dans un échantillon biologique.

La présente invention est davantage illustrée dans la partie expérimentale ci-dessous, qui n'en limite pas la portée.

### EXEMPLES

### Exemple 1 : mesure de la quantité d'ADN libre dans le sang de patientes avant de l'endométriose

### Matériels et Méthodes

### Cohorte

Un groupe de 32 femmes (16 sans antécédent d'endométriose et 16 avec des antécédents d'endométriose, traitées médicalement et/ou chirurgicalement) a fait l'objet de l'étude ci-dessous, avec leur consentement et l'accord du Comité d'éthique de l'Université de Sousse (Tunisie).

Les prélèvements sanguins ont été analysés par extraction de l'ADN libre et PCR quantitative pour connaitre la différence de concentration en ADN libre circulant entre les 2 groupes).

### Evaluation de l'ADN libre par PCR quantitative en temps réel

### Matériel

Les amorces suivantes ont été utilisées :
- RNP30_F : AGATTTGGACCTGCGAGCG (SEQ ID No : 1) et
- RNP30_R : GAAGCCGGGGCAACTCAC (SEQ ID No : 2).

Ces amorces amplifient une région de 86 paires de bases à cheval sur l'exon 1 et l'intron 2 du gène Homo sapiens ribonuclease P/MRP subunit p30, abrégé RPP30 (NM_006413, ENST00000371703.7).

Un thermocycleur en temps réel (LightCycLer® 480 Instrument II - Roche Life Science) a été utilisé selon les instructions du constructeur. Un "Master mix" contenant du SYBR™ Green (LightCycler® 480 SYBR Green I Master - Cat. No 04707516001) a été utilisé, ainsi qu'une Taq polymérase stable, sans activité enzymatique à température ambiante, qui est activée durant l'étape de dénaturation.

### Echantillons sanguins

Le sang a été prélevé dans des tubes SEC.

### Extraction du cfDNA

Afin de récupérer le surnageant, plusieurs centrifugations ont été effectuées de la manière suivante :
- une première centrifugation à 1600G pendant 10 minutes, puis récupération du surnageant (sérum).
- une deuxième centrifugation du sérum à 3200G pendant 20 min dans une centrifugeuse réfrigérée à 4°C. Cette centrifugation a permis d'éliminer tous les débris cellulaires.

Une fois extraits, les échantillons ont été congelés à -20°C. Des courbes de calibration ont été effectuées à l'aide d'ADN dont la concentration est connue.

L'extraction du cfDNA a été contrôlée avec des contrôles internes venant de serum de patients en diagnostic prénatal non invasif pour le diagnostic des trisomies 13,18,21 et XY.

### Gamme d'étalonnage et contrôles internes

Pour chaque série de PCR, une gamme étalonnage a été réalisée afin de doser précisément l'ADN libre dans les échantillons. Cette gamme de 8 points (50 ng/µL à 5.10⁻⁶ ng/µL), réalisée par dilutions successives, a permis d'obtenir une droite d'étalonnage dont les concentrations la plus haute et la plus basse encadraient les concentrations des échantillons.

Les échantillons à analyser sont passés au moins en duplicate et la moyenne des mesures a été calculée.

Sur chaque plaque, des contrôles négatifs (Master mix seul et Master mix + tampon) ont été effectués.

### Méthode de mesure de la concentration d'ADN libre

Un volume de 5 µL d'ADN extrait a été ajouté à 20 µL de 1 x Master mix contenant 0.5 µM de chaque amorce (en triplicat). L'amplification a consisté en une activation de 5 minutes à 95°C puis 35 cycles de dénaturation à 95°C pendant 10 secondes, d'hybridation à 59°C pendant 20 secondes et d'élongation à 72°C pendant 15 secondes, suivis d'une élongation finale de 5 minutes à 72°C.

Le calcul de la concentration se fait par régression linéaire avec une échelle de dilution de 10 fois, en triplicat, d'un ADN humain de concentration connue (en partant de 500'000 copies/réaction à 1'000 copies par réaction) et comparaison des Ct (*"Cycle threshold"*) selon les méthodes connues et décrites pour la PCR quantitative (qPCR).

A la fin de l'amplification, une courbe de fusion (Tm ou *"melting temperature"*) a été effectuée pour vérifier qu'un seul produit a été amplifié par PCR.

La température est portée à 95°C, puis abaissée à la température d'hybridation des amorces. Puis on l'augmente afin de désapparier les brins. La fluorescence est mesurée tout du long de l'hybridation.

Chaque produit d'amplification a une température de fusion qui dépend de sa composition en GC (Guanine, Cytosine), de la longueur de sa séquence nucléotidique, qui est également influencée par la concentration en sels (MgCl2) et par la concentration en SYBR™ Green. Les résultats, exprimés par la dérivée première de la courbe, montrent deux pics. Le premier pic correspond aux brins d'ADN amplifiés et le second à l'appariement des amorces entre elles.

### Résultats

Les résultats de la quantification du cfDNA par PCR en temps réel sont indiqués dans le Tableau 2 ci-dessous.

**Tableau 2 : Résultats de la Quantification du cfDNA par PCR en temps réel. Groupe Endométriose de 1 à 16. Groupe Témoin : de 17 à 32. Les échantillons sélectionnés pour la suite de l'analyse (exemple 2) ont été indiqués par une croix dans la dernière colonne.**

| Echantillons | copies génomiques/mL de plasma | SD [%] | ng d'ADN/mL de plasma | Echantillons sélectionnés |
|---|---|---|---|---|
| 1 | 19 325 | 0,5% | 66,64 | |
| 2 | 43 000 | 1,0% | 148,28 | |
| 3 | 248 750 | 4,2% | 857,76 | X |
| 4 | 76 750 | 0,7% | 264,66 | |
| 5 | 209 500 | 0,8% | 722,41 | X |
| 6 | 80250 | 0,8% | 276,72 | |
| 7 | 465 000 | 1,4% | 1 603,45 | X |
| 8 | 205 750 | 1,3% | 709,48 | X |
| 9 | 78 000 | 0,8% | 268,97 | |
| 10 | 158 750 | 3,3% | 547,41 | |
| 11 | 103 000 | 1,1% | 355,17 | |
| 12 | 395 000 | 1,3% | 1 362,07 | X |
| 13 | 40 750 | 0,2% | 140,52 | |
| 14 | 113 750 | 2,2% | 392,24 | |
| 15 | 79 000 | 3,0% | 272,41 | |
| 16 | 31 500 | 4,5% | 108,62 | |
| 17 | 23 750 | 0,4% | 81,90 | |
| 18 | 35 250 | 3,0% | 121,55 | |
| 19 | 228 500 | 1,5% | 787,93 | |
| 20 | 70 000 | 2,2% | 241,38 | X |
| 21 | 26250 | 2,0% | 90,52 | |
| 22 | 26250 | 0,0% | 90,52 | |
| 23 | 65250 | 1,7% | 225,00 | X |
| 24 | 3 8 500 | 0,2% | 132,76 | |
| 25 | 61 750 | 13,0% | 212,93 | |
| 26 | 84 500 | 2,9% | 291,38 | X |
| 27 | 41 750 | 1,3% | 143,97 | |
| 28 | 69 250 | 4,3% | 238,79 | X |
| 29 | 209 750 | 4,2% | 723,28 | |
| 30 | 332 500 | 6,6% | 1 146,55 | |
| 31 | 38 750 | 2,5% | 133,62 | |
| 32 | 75 000 | 0,0% | 258,62 | X |

En moyenne, le groupe endométriose contenait 56% de plus d'ADN libre que le groupe contrôle.

Cinq échantillons de chaque groupe ont été choisis pour réaliser un séquençage complet (*"Whole Genome Sequencing"*) et une analyse des profils de méthylation (méthylome). Les échantillons choisis dans le groupe endométriose contiennent en moyenne 4 fois plus d'ADN libre que les échantillons choisis dans le groupe témoin (1051 ng/mL versus 251 ng/mL).

Dans le groupe témoin, les échantillons 19, 29 et 30, présentant trois valeurs très élevées, sont susceptibles de correspondre à un état inflammatoire infra clinique, non diagnostiqué. Dans le groupe « endométriose », une valeur (échantillon 1) est très basse et trois valeurs sont dans les limites de la normale (échantillons 2, 13 et 16). Toutes les femmes recrutées dans le groupe endométriose avaient eu ou avaient un traitement médical en cours. Ces valeurs normales ou proches de la normale sont susceptibles de refléter l'efficacité du traitement.

Suite à l'amplification par PCR, les ADN ont été séparés par migration sur gel d'agarose, afin de mesurer la taille des fragments d'ADN dans le cfDNA. Cela a permis d'observer la taille des fragments d'ADN, allant de 1353 à 72 pb.

### Exemple 2 : Analyse du profil de méthylation différentiel du cfDNA

Le cfDNA de 5 femmes de chacun des deux groupes (voir Tableau 2) a été soumis à une étude génomique complète (WGS) avec, pour chacun des gènes identifiés, son statut de méthylation. Cette étude a été réalisée par la plateforme de recherche ACOBIOM à Montpellier.

### Matériels et Méthodes

### Préparation des ADNs

Le traitement au bisulfite des ADNs a été réalisé selon le protocole ROCHE (KAPA Library Préparation Kit Illumina®, 07138008001), en supprimant l'étape de fragmentation de l'ADN, inutile puisque l'ADN circulant est naturellement fortement fragmenté.

La ligation des adaptateurs A et B a été réalisée selon le protocole ROCHE (SeqCap® Adapter Kit A et/ou B, 07141530001), en modifiant l'étape de ligation des adaptateurs : 30 min à 20°C puis une nuit à 16°C.

Enfin, la conversion au bisulfite a été réalisée selon le protocole ZYMO (EZ DNA Methylation-Lightning™ kit, D5030).

### Séquençage des ADNs

Les librairies d'ADN sont préparées selon le protocole du kit ROCHE (Capture d'ADN avec le kit SeqCap® Epi Enrichment System, 05634261001) en modifiant le nombre de cycles PCR avant capture : 14 cycles en PCR pré-capture.

Avant séquençage, les ADN ont été vérifiés et dosés sur la plateforme PERKINELMER (Labchip® GX). Le séquençage a été réalisé selon le protocole ILLUMINA sur la plateforme NextSeq®.

### Traitement Bioinformatique

### Étapes du traitement

L'analyse des données de méthylation, obtenues par séquençage sur plateforme Illumina à partir du kit *"Roche NimbleGen SeqCap*® *Epi target enrichment",* a été réalisée à partir de logiciels et de bases de données libres. Les données ont été analysées selon le protocole fourni par Roche.

Typiquement, l'analyse de méthylation a suivi ces différentes étapes :

### (i) Contrôle de la qualité des séquences (logiciel FastQC)

Mesure et rapporte la qualité des bases pour chaque fichier, pour estimer et valider la qualité du séquençage.

### (ii) Nettoyage des séquences (Logiciel Trimmomatic v 0.36)

Nettoie les séquences Illumina en suivant certains critères :
- Supprime les séquences de mauvaise qualité
- Reconnaît et élimine les séquences artificielles (adaptateurs + code-barres)
- Supprime les séquences trop courtes

### (iii) Alignement des séquences sur le génome de référence (logiciel BSMAP v2.90)

Aligne chaque séquence traitée au bisulfite sur le génome de référence : génome Homo sapiens version GRCh37 (hg19).

### (iv) Triage et suppression des doublons générés par la PCR (logiciels Samtools v1.8, Bamtools v2.4.1 & Picard/MarkDuplicates v2.8.1)

Lors du traitement au bisulfite, les bases C non méthylées sont transformées en T. Cela a pour conséquence que les deux brins d'ADN ne sont plus complémentaires. Lors de la PCR, l'amplification de ces brins va générer artificiellement de nouveaux brins complémentaires qu'il faut supprimer.

### (v) Mesure du pourcentage de méthylation (logiciel BSMAP v 2.90)

Détermine le pourcentage de méthylation pour chaque base C, pour chacun des fichiers.

Ces résultats sont utilisés pour l'analyse différentielle des régions méthylées (logiciel methylKit R) (voir l'étape de Traitement Statistique)

### (vi) Estimation du taux d'efficacité de conversion par le bisulfite (BSMAP v 2.90)

Pour déterminer l'efficacité de la conversion au bisulfite, on mesure le nombre de C convertis pour une molécule d'ADN qui n'est pas méthylée. Le kit utilisé contient une séquence contrôle, de l'ADN de phage Lambda (Identifiant GenBank : NC_001416), qui est ajouté à l'échantillon. Le kit contient également les sondes de capture du phage Lambda.

Après l'étape d'alignement, on mesure le nombre de C transformés en T sur les séquences spécifiques du phage Lambda. Ce taux doit être proche de 100 %.

### Contrôle des librairies de séquençage

Le logiciel FastQC a été utilisé pour effectuer les contrôles de qualité sur les données de séquençage à haut débit. Pour chaque fichier de séquençage, un rapport a été généré. Le logiciel MultiQC a regroupé ces résultats de contrôle de qualité pour générer un résumé dans un rapport unique (en html).

### Contrôle du traitement au bisulfite

Pour déterminer l'efficacité de la conversion, le nombre de C convertis sur l'ADN (non-méthylé) du phage Lambda a été mesuré. Tous les échantillons ont été testés et le taux moyen de conversion au bisulfite est supérieur à 99,4 %. Ces résultats permettent de considérer que l'étape de conversion au bisulfite s'est correctement déroulée.

### Résultats

L'analyse des échantillons a permis d'identifier 91 gènes hyperméthylés (Tableau 3) et 66 gènes hypométhylés (Tableau 4) chez les femmes endométriosiques.

**Tableau 3 : gènes hyperméthylés chez les femmes endométriosiques**

| **Nom** | **description** | **Identifiant GenBank** | **Différentiel de méthylation** |
|---|---|---|---|
| CALD1 | *caldesmon 1* | NM_033140 | **-63,7** |
| RRP1 | *ribosomal RNA processing 1* | NM_003683 | **-54,3** |
| FN1 | *fibronectin 1* | NM_212482 | **-54,3** |
| FAM87B | *family with sequence similarity 87 member B* | NR_103536 | **-52,5** |
| TCEAL6 | *transcription elongation factor A like 6* | NM_001006938 | **-50,7** |
| RPL29P2 | *ribosomal protein L29 pseudogene 2* | NR_002778 | **-50,5** |
| ATP11A-AS1 | *ATP11A antisense RNA 1* | NR_046661 | **-50,0** |
| DIP2C | *disco interacting protein 2 homolog C* | NM_014974 | **-47,0** |
| SLCO2B1 | *solute carrier organic anion transporter family member 2B1* | NM_007256 | **-46,5** |
| RMI2 | *RecQ mediated genome instability 2* | NM_152308 | **-46,4** |
| CSTF2T | *cleavage stimulation factor subunit 2 tau variant* | NM_015235 | **-46,0** |
| LOC283177 | *uncharacterized LOC283177* | NR_033852 | **-44,5** |
| FXYD2 | *FXYD domain containing ion transport regulator 2* | NM_001680 | **-44,0** |
| ZNF33B | *zinc finger protein 33B* | NM_006955 | **-42,2** |
| MIR3170 | *microRNA 3170* | NR_036129 | **-42,1** |
| LINC01007 | *long intergenic non-protein coding RNA 1007* | NR_103749 | **-41,4** |
| RNU6-2 | *RNA, U6 small nuclear 2* | NR_125730 | **-40,9** |
| REEP3 | *receptor accessory protein 3* | NM_001001330 | **-40,9** |
| ALG10 | *ALG10, alpha-1,2-glucosyltransferase* | NM_032834 | **-40,8** |
| OR2AG1 | *olfactory receptor family 2 subfamily AG member 1* | NM_001004489 | **-40,5** |
| IQGAP2 | *IQ motif containing GTPase activating protein 2* | NM_006633 | **-40,2** |
| TSPAN17 | *tetraspanin 17* | NM_012171 | **-39,7** |
| MIR4693 | *microRNA* 4693 | NR_039842 | **-39,5** |
| HYOU1 | *hypoxia up-regulated 1* | NM_006389 | **-39,0** |
| TLR4 | *toll like receptor 4* | NM_138554 | **-38,8** |
| LINC00689 | *long intergenic non-protein coding RNA* 689 | NR_024394 | **-38,8** |
| PCOTH | *upstream in-frame stop codon* | NM_001135816 | **-38,7** |
| MLN | *motilin* | NM_001040109 | **-38,6** |
| ADGRL3 | *adhesion G protein-coupled receptor L3* | NM_015236 | **-37,9** |
| LINC01939 | *long intergenic non-protein coding RNA 1939* | NR_110179 | **-37,6** |
| ALG1L2 | *upstream in-frame stop codon* | NM_001136152 | **-37,5** |
| IL6 | *interleukin 6* | NM_000600 | **-37,1** |
| PHYKPL | *5-phosphohydroxy-L-lysine phospho-lyase* | NR_103508 | **-36,8** |
| ZSWIM4 | *zinc finger SWIM-type containing 4* | NM_023072 | **-36,6** |
| VIRMA | *vir like m6A methyltransferase associated* | NM_015496 | **-35,8** |
| RPS6KA2 | *ribosomal protein S6 kinase A2* | NM_001006932 | **-34,8** |
| FAM25A | *family with sequence similarity 25 member A* | NM_001146157 | **-34,8** |
| MKRN1 | *makorin ring finger protein 1* | NR_117084 | **-34,5** |
| ADARB2 | *adenosine deaminase RNA specific B2 (inactive)* | NM_018702 | **-34,5** |
| MGC16025 | *uncharacterized LOC85009* | NR_026664 | **-34,5** |
| PFKP | *phosphofructokinase, platelet* | NM_001242339 | **-33,2** |
| FMO2 | *flavin containing monooxygenase 2* | NM_001460 | **-33,2** |
| INSIG1 | *insulin induced gene 1* | NM_198337 | **-33,1** |
| LINC00824 | *long intergenic non-protein coding RNA 824* | NR_121672 | **-32,7** |
| ABR | *active BCR-related* | NM_001256847 | **-32,4** |
| ROR2 | *receptor tyrosine kinase like orphan receptor 2* | NM_004560 | **-32,2** |
| MRPL3 | *mitochondrial ribosomal protein L3* | NM_007208 | **-31,6** |
| VANGL1 | *VANGL planar cell polarity protein 1* | NM_001172412 | **-31,5** |
| BTD | *biotinidase* | NM_001281723 | **-31,0** |
| AFF3 | *AF4*/*FMR2 family member 3* | NM_002285 | **-30,9** |
| DNAH9 | *dynein axonemal heavy chain 9* | NM_001372 | **-30,8** |
| LINC02209 | *long intergenic non-protein coding RNA 2209* | NR_024473 | **-30,6** |
| LOC100506422 | *upstream in-frame stop codon* | NM_001004352 | **-30,3** |
| CAP2 | *upstream in-frame stop codon* | NM_006366 | **-30,1** |
| FMNL2 | *formin like 2* | NM_052905 | **-30,1** |
| TMEM19 | *transmembrane protein 19* | NM_018279 | **-29,9** |
| MLLT1 0 | *MLLT10, histone lysine methyltransferase DOT1L cofactor* | NM_001195626 | **-29,6** |
| ZNF438 | *zinc finger protein 438* | NM_182755 | **-29,5** |
| LINC01192 | *long intergenic non-protein coding RNA 1192* | NR_033945 | **-29,2** |
| SCHIP1 | *schwannomin interacting protein 1* | NM_001197109 | **-29,2** |
| RCBTB1 | *RCC1 and BTB domain containing protein 1* | NM_018191 | **-29,1** |
| LOC101929420 | *uncharacterized LOC101929420* | NR_110870 | **-29,1** |
| TSPAN33 | *tetraspanin 33* | NM_178562 | **-29,0** |
| ENGASE | *endo-beta-N-acetylglucosaminidase* | NM_001042573 | **-28,9** |
| TMPRSS6 | *transmembrane serine protease 6* | NM_153609 | **-28,8** |
| NKD2 | *NKD inhibitor of WNT signaling pathway 2* | NM_001271082 | **-28,4** |
| IGSF11 | *immunoglobulin superfamily member 11* | NM_001015887 | **-28,1** |
| LOC100134317 | *uncharacterized LOC100134317* | NR_029389 | **-28,1** |
| FGFR2 | *fibroblast growth factor receptor 2* | NR_073009 | **-27,9** |
| LOC100134317 | *uncharacterized LOC100134317* | NR_029389 | **-27,8** |
| LOC101927972 | *uncharacterized LOC101927972* | NR_125848 | **-27,8** |
| TPRG1 | *tumor protein p63 regulated 1* | NM_198485 | **-27,8** |
| DUSP21 | *dual specificity phosphatase 21* | NM_022076 | **-27,7** |
| MIR4644 | *microRNA 4644* | NR_039787 | **-27,5** |
| FOXO4 | *forkhead box O4* | NM_005938 | **-27,0** |
| LTBP1 | *upstream in-frame stop codon* | NM_000627 | **-26,8** |
| LINC01164 | *long intergenic non-protein coding RNA 1164* | NR_038365 | **-26,6** |
| FSTL1 | *follistatin like 1* | NM_007085 | **-26,4** |
| LOC100287846 | *uncharacterized LOC100287846* | NR_037168 | **-26,4** |
| CLMN | *calmin* | NM_024734 | **-26,3** |
| CNIH3 | *cornichon family AMPA receptor auxiliary protein 3* | NM_152495 | **-26,3** |
| MICUS3 | *mitochondrial calcium uptake family member 3* | NM_181723 | **-26,3** |
| KRTAP19-8 | *keratin associated protein 19-8* | NM_001099219 | **-25,9** |
| LOC101927620 | *uncharacterized LOC101927620* | NR_110062 | **-25,9** |
| CLMN | *calmin* | NM_024734 | **-25,8** |
| ARL5B | *ADP ribosylation factor like GTPase 5B* | NM_178815 | **-25,7** |
| TMEM44-AS1 | *TMEM44 antisense RNA 1* | NR_047573 | **-25,7** |
| FOXO4 | *forkhead box O4* | NM_001170931 | **-25,7** |
| JPH3 | *junctophilin 3* | NM_001271605 | **-25,6** |
| FAM210B | *family with sequence similarity 210 member B* | NM_080821 | **-25,5** |
| CLMN | *calmin* | NM_024734 | **-25,3** |

**Tableau 4 : gènes hypométhylés chez les femmes endométriosiques**

| **Nom** | **Description** | **Identifiant GenBank** | **Différentiel de méthylation** |
|---|---|---|---|
| NOP56 | NOP56 ribonucleoprotein | NR_027700 | **25,1** |
| FREM2 | FRAS1 related extracellular matrix protein 2 | NM_207361 | **25,3** |
| NT5C2 | 5'-nucleotidase, cytosolic II | NM_012229 | **25,5** |
| DLGAP2 | DLG associated protein 2 | NM_004745 | **25,6** |
| NAV1 | neuron navigator 1 | NM_020443 | **25,8** |
| C22orf42 | chromosome 22 open reading frame 42 | NM_001010859 | **26,2** |
| SOD3 | superoxide dismutase 3 | NM_003102 | **26,4** |
| C3 | complement C3 | NM_000064 | **27,0** |
| PGS1 | phosphatidylglycerophosphate synthase 1 | NR_111989 | **27,5** |
| UBE3A | ubiquitin protein ligase E3A | NM_130839 | **27,7** |
| FOXL1 | forkhead box L1 | NM_005250 | **28,0** |
| SEPT-1 | septin 1 | NM_018243 | **28,1** |
| MIR4655 | microRNA 4655 | NR_039799 | **28,2** |
| TSGA13 | testis specific 13 | NM_052933 | **28,3** |
| MY05C | myosin VC | NM_018728 | **28,6** |
| LINC00211 | long intergenic non-protein coding RNA 211 | NR_110011 | **28,8** |
| PAH | phenylalanine hydroxylase | NM_000277 | **29,1** |
| PDE3A | phosphodiesterase 3A | NM_000921 | **29,1** |
| SGIP1 | upstream in-frame stop codon | NM_032291 | **29,3** |
| LINCR-0001 | uncharacterized LINCR-0001 | NR_120604 | **29,3** |
| COX6C | cytochrome c oxidase subunit 6C | NM_004374 | **29,4** |
| MIR6133 | microRNA 6133 | NR_106749 | **30,1** |
| LOC389602 | uncharacterized LOC389602 | NM_001291913 | **30,4** |
| BRSK2 | BR serine/threonine kinase 2 | NM_001256629 | **30,6** |
| MIR4277 | microRNA 4277 | NR_036240 | **30,6** |
| RPS27A | ribosomal protein S27a | NM_001135592 | **30,8** |
| FAM133B | family with sequence similarity 133 member B | NR_109929 | **31,2** |
| THNSL2 | threonine synthase like 2 | NM_018271 | **31,2** |
| LINC01968 | long intergenic non-protein coding RNA 1968 | NR_037891 | **31,5** |
| GUCA1C | guanylate cyclase activator 1C | NM_005459 | **31,6** |
| MIR4251 | microRNA4251 | NR_036215 | **31,9** |
| LOC101928708 | uncharacterized LOC101928708 | NR_110939 | 31,9 |
| CRAT-PLPP7 | phospholipid phosphatase 7 (inactive) | NM_032728 | 31,9 |
| CSAD | cysteine sulfinic acid decarboxylase | NM_001244705 | **32,0** |
| MN1 | MN1 proto-oncogene, transcriptional regulator | NM_002430 | **32,1** |
| CBR4 | carbonyl reductase 4 | NM_032783 | **32,5** |
| LINC02421 | long intergenic non-protein coding RNA 2421 | NR_110063 | **32,6** |
| LOC101928861 | uncharacterized LOC101928861 | NR_120513 | **33,0** |
| LOC100129203 | uncharacterized LOC100129203 | NR_110295 | **33,7** |
| FAM86EP | family with sequence similarity 86 member E | NR_024253 | **34,3** |
| TAF8 | TATA-box binding protein associated factor 8 | NM_138572 | **34,9** |
| MIR3666 | microRNA 3666 | NR_037439 | **34,9** |
| LOC100129534 | small nuclear ribonucleoprotein polypeptide N pseudogene | NR_024489 | **35,3** |
| ZNF496 | zinc finger protein 496 | NM_032752 | **35,8** |
| GRAMD1B | GRAM domain containing 1B | NM_001286564 | **36,2** |
| AZIN1 | antizyme inhibitor 1 | NM_148174 | **36,2** |
| FAM209B | family with sequence similarity 209 member B | NM_001013646 | **36,6** |
| MIR4251 | microRNA 4251 | NR_036215 | **36,9** |
| WDTC1 | WD and tetratricopeptide repeats 1 | NM_015023 | **37,4** |
| EPS8L1 | EPS8 like 1 | NM_133180 | **37,6** |
| DNAH5 | dynein axonemal heavy chain 5 | NM_001369 | **38,1** |
| SLC37A2 | solute carrier family 37 member 2 | NM_198277 | **38,3** |
| FASTKD1 | FAST kinase domains 1 | NR_104020 | **38,5** |
| LYZL1 | lysozyme like 1 | NM_032517 | **38,6** |
| FZD10 | frizzled class receptor 10 | NM_007197 | **39,5** |
| FAM187B | family with sequence similarity 187 member B | NM_152481 | **39,9** |
| CD81 | CD81 molecule | NM_001297649 | **40,1** |
| STAU2-AS1 | STAU2 antisense RNA 1 | NR_038406 | **40,4** |
| TDRD5 | tudor domain containing 5 | NM_001199091 | **40,5** |
| USP1 | ubiquitin specific peptidase 1 | NM_003368 | **42,3** |
| ACVR2A | activin A receptor type 2A | NM_001616 | **42,5** |
| FBXO38 | F-box protein 38 | NM_001271723 | **47,3** |
| FASN | fatty acid synthase | NM_004104 | **47,7** |
| MKRN9P | makorin ring finger protein 9, pseudogene | NR_033410 | **50,2** |
| PCCA-AS1 | PCCA antisense RNA 1 | NR_047686 | **50,7** |
| RP9 | RP9, pre-mRNA splicing factor | NM_203288 | **52,8** |

Les gènes méthylés différentiellement (DMG) ont ensuite été classés par ontologie à l'aide du logiciel *Ingenuity Pathway Analysis* (IPA), permettant d'identifier les voies de signalisation et métaboliques, les réseaux moléculaires et les fonctions biologiques les plus pertinents pour une liste de gènes, afin de rechercher des processus biologiques potentiels, des voies de signalisation et les relations réciproques entre les gènes du réseau.

Des réseaux de ces gènes ont été générés en fonction de leur connectivité. Un score de réseau a été calculé sur la base de la distribution hypergéométrique et calculé à l'aide du test exact de Fisher (la valeur p <0,05 a été considérée comme significative).

Les Figures 1 à 5 montrent certains des réseaux générés.

Le premier réseau (Figure 1), montre que plusieurs gènes hyperméthylés sont impliqués dans les maladies inflammatoires et de la signalisation, ainsi que dans les interactions entre cellules : on note l'importance de IL6, pilier de la régulation inflammatoire et de CALD 1, FN1, TLR4, JPH3 et INSIG1.

Parmi les gènes hyperméthylés, plusieurs régulateurs amont des voies WNT5A, IFN béta et du récepteur aux oestrogènes ont été identifiés (Tableau 5)

**Tableau 5 : Principaux gènes hyperméthylés : régulateurs en amont (WNT5A, IFN Béta, récepteur aux oestrogènes)**

| **Gènes : initiales** | **Noms** | **Différentiel de méthylation** | **Localisation** | **Types(s)** |
|---|---|---|---|---|
| FGFR2 | Fibroblast growth factor | 27,873 | Membrane plasmatique | Kinase |
| FN1 | Fibronectine 1 | 54,264 | Espace extracellulaire | Enzyme |
| IL6 | Interleukine 6 | 37,066 | Espace extracellulaire | Cytokine |
| ROR2 | Recepteur tyrosine kinase | 32,170 | Membrane plasmatique | Kinase |
| TLR4 | Toll like receptor 4 | 38,821 | Membrane plasmatique | Récepteur transmembranaire |

A l'inverse, le réseau de gènes hypométhylés est enrichi en gènes associés au métabolisme des glucides et à la production d'énergie (FASN, NT5C2), et au cycle cellulaire (UBE3A, FASN, BRSK2, SOD3, PDE3A) (tableaux 6 à 8).

La signature du développement embryonnaire apparaît notamment dans ce réseau. Sont inclus ACVR2A, FZD10, CASAD, COX6C et USP1.

**Tableau 6 : gènes hypométhylés associés au métabolisme des glucides**

| **Catégories** | **Fonctions** | **p-Value** | **Molécules** |
|---|---|---|---|
| Métabolisme des carbohydrates, Production d'énergie | Oxydation des carbohydrates | 2,80^{E}-06 | FASN, NT5C2 |
| Métabolisme des carbohydrates, Production d'énergie | Oxidation du D-glucose | 2,00E-04 | NT5C2 |
| Métabolisme des carbohydrates | Incorporation du glycogène | 4,79^{E}-06 | NT5C2 |
| Métabolisme des carbohydrates, Petites molécules | Incorporation du D-glucose | 9,55E-03 | NT5C2 |
| Métabolisme des carbohydrates, Transport moléculaire | Transport du glucose-6 phosphate | 9,55E-03 | SL37A2 |
| Métabolisme des carbohydrates, Transport moléculaire | Transport des monosaccharide | 1,00E-02 | NT5C2, SLC37A2 |
| Métabolisme des carbohydrates, Métabolisme des médicaments | Interaction avec l'heparane sulfate de protéoglycane et le collagène. Anti-oxydation | 1,19E-02 | S0D3 |

**Tableau 7 : gènes hypométhylés associés à la production d'énergie**

| **Catégories** | **Fonctions** | **p-Value** | **Molécules** |
|---|---|---|---|
| Métabolisme des carbohydrates, Production d'énergie | Oxydation des carbohydrates | 2,80E-06 | FASN, NT5C2 |
| Métabolisme des carbohydrates, Production d'énergie | Oxydation du D-glucose | 2,00E-04 | NT5C2 |
| Réplication, Recombinaison, et Réparation de l'ADN | Oxydation du NADPH | 1,43E-02 | FASN |
| Production d'énergie, Métabolisme des lipides | Oxydation de l'acide palmitique | 2,61 E-02 | NT5C2 |

**Tableau 8 : gènes hypométhylés associés aux fonctions inter cellulaires**

| **Catégories** | **Fonctions** | **p-Value** | **Molécules** |
|---|---|---|---|
| Cycle cellulaire | Sénescence des lignées cellulaires de lymphomes | 2,40E-03 | UBE3A |
| Cycle cellulaire | Arrêt en G2/M de la transition de phase du mélanome | 7,17E-03 | BRSK2 |
| Cycle cellulaire | Sénescence de lignées cellulaires hépatiques | 7,17E-03 | FASN |
| Cycle cellulaire, cancer | Arrêt de la progression du cycle cellulaire de cellules tumorales | 1,90E-02 | FASN |
| Cycle cellulaire | Arrêt en phase G2 de lignées de cellules tumorales | 4,38E-02 | BRSK2, FASN |
| Cycle cellulaire | Arrêt en phase G2 de lignées de cellules de cancer colorectal | 4,92E-02 | FASN |

Le réseau de la Figure 2 illustre les voies de conduction transmembranaire, notamment l'implication du récepteur du TGF Béta et des protéines transmembranaires Frizzled (Fz), famille de récepteurs couplés aux protéines G (RCPG) qui tiennent notamment un rôle dans la voie de signalisation Wnt.

Le réseau de la Figure 3 montre les liens entre les gènes hypométhylés chez les femmes endométriosiques, impliqués dans la mort et la survie cellulaires, le mouvement cellulaire et le cycle cellulaire.

En filtrant et en croisant les voies de régulation, les voies métaboliques, la production d'énergie et la réplication de l'ADN avec sa réparation, une liste de 15 gènes seulement, fortement impliqués dans l'endométriose, a été retenue (10 hyperméthylés et 5 hypométhylés) (Tableau 9).

**Tableau 9 : 15 gènes (10 hyperméthylés et 5 hypométhylés) fortement impliqués dans l'endométriose**

| **Gènes** | **Différentiel de méthylation** |
|---|---|
| **Hyperméthylés** | |
| CALD1 | -63,726 |
| RRP1 | -54,317 |
| FN1 | -54,264 |
| FAM87B | -52,474 |
| TCEAL6 | -50,702 |
| RPL29P2 | -50,481 |
| ATP11A-AS1 | -49,984 |
| DIP2C | -47,044 |
| SLCO2B1 | -46,491 |
| RMI2 | -46,429 |

| **Hypométhylés** | |
|---|---|
| FBXO38 | 47,310 |
| ACVR2A | 42,478 |
| USP1 | 42,336 |
| TDRD5 | 40,465 |
| STAU2-AS1 | 40,417 |

Le réseau de la Figure 4 montre l'interrelation entre les gènes hyper et hypo-méthylés dans le développement du tissu conjonctif et le développement cellulaire fonctionnel. Les flèches représentent les cibles essentielles.

Le réseau de la Figure 5 montre l'importance du récepteur aux oestrogènes (crucial dans le développement de l'endométriose). Les gènes retenus (36 parmi les 91 hyperméthylés) sont signalés par une étoile. Ceux retenus parmi les 66 gènes hypométhylés (n= 26) sont signalés par une forme de nuage.

Ainsi, une grande partie des DMG est impliquée dans le métabolisme, la production d'énergie et la réparation et la réplication de l'ADN. En particulier, il a été observé que les voies de signalisation Wnt et de signalisation TGFbeta sont associées de manière significative aux gènes hypométhylés.

Parmi les 36 gènes hyperméthylés (CALD1, RRP1, FN1, FAM87B, TCEAL6, RPL29P2, ATP11A-AS1, DIP2C, SLCO2B1, RMI2, MIR3170, LINC01007, TSPAN17, MIR4693, HYOU1, TLR4, ADGRL3, IL6, VIRMA, MKRN1, INSIG1, ROR2, MRPL3, FMNL2, TMEM19, ZNF438, LINC01192, RCBTB1, TSPAN33, NKD2, FGFR2, TPRG1, MIR4644, FOXO4, FSTL1, CLMN) et les 26 gènes hypométhylés (NT5C2, NAV1, SOD3, C3, UBE3A, MIR4655, MYO5C, COX6C, MIR6133, BRSK2, MIR4277, MIR4251, MN1, MIR3666, AZIN1, MIR4251, SLC37A2, FZD10, STAU2-AS1, TDRD5, USP1, ACVR2A, FBXO38, FASN, MKRN9P, PCCA-AS1) dont les fonctions régulent les processus de survie et de développement des cellules endométriosiques, les inventeurs ont donc sélectionné 15 gènes qui sont particulièrement impliqués : 10 hyperméthylés (CALD1, RRP1, FN1, FAM87B,TCEAL6, RPL29P2, ATP11A-AS1, DIP2C, SLCO2B1, RMI2) et 5 hypométhylés (FBX038, ACVR2A, USP1, TDRD5, STAU2-AS1). Cette liste a été sélectionnée à partir des données brutes soumises à des networks qui rendent comptent des principaux gènes qui interagissent, et de leur statut de méthylation. Une étude sur une cohorte plus importante peut, le cas échéant, entraîner des modifications ou compléments à cette liste.

### Discussion

Les résultats de cette étude sérique associent l'excès d'ADN libre et un panel restreint de 15 gènes dont les variations de méthylation jouent un rôle essentiel dans la croissance et le développement de l'endométriose. C'est, à ce jour, une étude unique, dans le sérum, non invasive et dont les données permettent d'évoquer le diagnostic de la maladie et de porter un pronostic.

L'endométriose, affection fréquemment invalidante, a été décrite successivement comme une maladie hormonale, une maladie immunitaire, une maladie génétique et également une maladie provoquée par une exposition à des facteurs environnementaux.

L'impact économique de l'endométriose résulte du retard au diagnostic de l'endométriose, en particulier chez les jeunes femmes en période d'activité génitale qui sont traitées trop tardivement. La laparoscopie reste le *"gold standard"* du diagnostic. L'examen anatomopathologique et immuno-histochimique des cellules endométriosiques a, jusqu'à ce jour, permis d'authentifier des gènes divers en fonction du stade de l'endométriose, des tissus atteints et de l'évolutivité de la maladie. L'hétérogénéité des résultats est due à l'hétérogénéité des tissus et groupes étudiés. De nombreuses anomalies, notamment des cytokines (interleukine 1: IL1, pilier de l'immunité, l'homeobox A10 (HOXA 10) et une expression accrue de l'interleukine 6 (IL6) et du BCL 2 (facteur apoptotique), ont été décrites dans les cellules endométriosiques, et des gènes pertinents y ont été identifiés. (Petracco *et al.* 2011 ; Ahn *et al.,* 2016).

Le cfDNA circulant a été proposé comme marqueur plasmatique potentiel du cancer mais aussi de l'endométriose, minime ou moyenne (Zachariah *et al.,* 2009) avec une sensibilité à 70% et une spécificité à 87%.

Reflet du stress oxydatif, les taux plasmatiques de cfDNA sont significativement plus élevés chez la femme infertile (EP2879696B1 ; Hazout *et al.,* 2018).

Les processus inflammatoires et de stress cellulaire étant importants dans les états d'endométriose, il n'est pas surprenant de retrouver des taux plasmatiques élevés. Cependant, parce que l'endométriose peut être découverte chez des femmes asymptomatiques, la voie biochimique de la stimulation nerveuse par la libération de certaines molécules (Laux-Biehlmann *et al.,* 2015) n'est pas valable ; ce qui suggère que l'inflammation provoquée par les foyers ectopiques peut ne pas être la seule cause de douleur pelvienne.

L'endométriose est en effet une maladie inflammatoire chronique dans laquelle l'inflammation, stérile, induit par le dépôt de fragments d'endomètres cycliques, des perturbations épigénétiques.

Les phénotypes épigénétiques sont conférés par des processus nucléaires tels que la méthylation de l'ADN et les modifications de la chromatine, via des microARN et des ARN non codants à double brin, qui sont interconnectés et peuvent opérer ensemble pour établir et maintenir des états d'activités géniques spécifiques dans des cellules normales (Wang *et al.,* 2015) (Wang *et* al., 2016). La modification épigénétique la mieux comprise est la méthylation de l'ADN de la position 5 sur les atomes de carbone du noyau pyrimidine des cytosines au niveau des dinucléotides cytosine - guanosine (sites CpG). La méthylation de l'ADN convertit la cytosine en 5-méthylcytosine qui est couplée correctement avec une guanosine complémentaire. Malgré la tendance générale à la méthylation des CpG dans tout le génome, les sites CpG des îlots CpG, et en particulier ceux associés aux promoteurs de gènes, sont généralement peu méthylés, ce qui leur permet de participer à la transcription active du gène.

Lorsque les îlots CpG du promoteur sont hyperméthylés, le gène devient généralement silencieux. Les îlots sont relativement stables, mais réversibles, et les ADN méthyltransférases (DNMT) de maintenance assurent l'héritage épigénétique lors de la réplication de l'ADN. Inversement, les promoteurs peu méthylés sont associés à l'activation des gènes au niveau de la transcription et on estime qu'ils représentent environ 20-30% du génome humain.

Dans la mise en oeuvre de l'invention, l'étude sérique du cfDNA et le profil de méthylation de 15 gènes candidats permettent de confirmer le diagnostic de l'endométriose.

L'expression aberrante de l'ADN méthyltransférase, (DNMT) qui attache un groupe méthyle en position 5 des atomes de carbone des bases cytosines dans l'îlot CpG de la région promotrice et fait taire l'expression génique correspondante, a également été démontrée dans l'endométriose (Naqvi *et al.,* 2014). Les preuves accumulées suggèrent que diverses aberrations épigénétiques jouent des rôles précis dans la pathogenèse de l'endométriose (*Cho et al.,* 2015). Des études récentes ont décrit des gènes hyper ou hypométhylés sur des cellules endométriosiques explorées en immunohistochimie sur la base de gènes connus pour être impliqués dans la stéroïdogénèse ou l'expression de gènes présents dans le tissu endométrial (Vassilopoulou *et al.* 2019).

Dans le contexte sanguin, le degré d'hyperméthylation des gènes candidats (blocage de transcription) reflète l'intensité et/ou le potentiel évolutif de l'affection, éventuellement contrecarrée par d'autres gènes hypométhylés actifs.

La présente invention consiste à établir dans le sang circulant la coexistence d'un syndrome de stress oxydatif exprimé par le cfDNA sérique et le statut de méthylation de gènes impliqués dans tous les mécanismes favorisant la croissance et le développement de l'endométriose : régulateurs en amont des fonctions cellulaires et moléculaires (organisation, protéomique, signalisation et interaction intercellulaire).

Des 158 gènes isolés (92 hyperméthylés et 66 hypométhylés), un panel de 62 gènes a été reconnu dans le développement de l'endométriose : 36 hyperméthylés et 26 hypométhylés.

A partir de ce panel, après une analyse bioinformatique, une grande partie des gènes faiblement méthylés est impliquée dans le métabolisme, la production d'énergie et la réparation et la réplication de l'ADN. Les voies de signalisation Wnt et de signalisation TGFbeta étaient associées de manière significative aux gènes hypométhylés enrichis en gènes associés au métabolisme des glucides et à la production d'énergie (SEPT-1, FASN, NT5C2), au décès/survie et au cycle cellulaire (UBE3A, FASN, BRSK2, SOD3, PDE3A). Le CRAT est sur le chromosome 9q34.11. Ce gène a été aussi reconnu à la place du PLPP7 : 9q34.13, qui se trouve sur le même chromosome.

La voie de signalisation IL6 est associée de manière significative aux gènes hyperméthylés. Les réseaux fonctionnels pour la morphologie cellulaire, l'assemblage cellulaire, l'inflammation et la signalisation intercellulaire sont liés aux gènes hyperméthylés (FN1, IL6, TLR4, FGFR2, RCBTB1, IFN beta, CALD1 et ROR2).

De plus, FN1, CALD1 et JPH3 montrent une connexion avec le récepteur aux oestrogènes.

Des études récentes ont montré des niveaux altérés de l'expression du gène CALD1 (codant pour la protéine Caldesmon) dans les lésions d'endométriose (Meola *et al.,* 2013). La fibronectine est impliquée dans les processus d'adhésion et de migration des cellules, la cicatrisation des plaies, la coagulation du sang, la défense de l'hôte et les métastases. Le gène FN1 a trois régions sujettes à un épissage alternatif, avec la possibilité de produire 20 variants de transcription différents, dont au moins un code pour une isoforme qui subit un traitement protéolytique. L'anastelline se lie à la fibronectine et induit la formation de fibrilles. Ce polymère de fibronectine, appelé superfibronectine, présente des propriétés adhésives améliorées. L'anastelline et la superfibronectine inhibent la croissance tumorale, l'angiogenèse et les métastases. JPH3 et DIP2C sont exprimés dans le système nerveux. L'IL6 induit aussi la différenciation des cellules nerveuses

FASN est fusionnée avec le récepteur des oestrogènes alpha (ER-alpha), SOD3 protège l'espace extracellulaire des effets toxiques des dérivés actifs de l'oxygène en convertissant les radicaux superoxydes en peroxyde d'hydrogène et oxygène.

Une récente méta-analyse (Sapkota *et al.* 2017) a identifié de nouveaux loci associés avec l'endométriose, mettant en évidence les gènes clés impliqués dans le métabolisme hormonal (dont le FN1 et l'ESR1).

En conclusion, l'étude présentée ici est le premier travail montrant une augmentation significative de l'ADN libre dans le sérum des femmes souffrant de douleurs suspectes d'endométriose, associé à un panel de gènes hyper et hypométhylés régulant l'expression et le développement de l'endométriose.

La présente invention ouvre donc des perspectives de diagnostic précoce de l'endométriose par le taux de cfDNA sérique en excès, le dit diagnostic étant associé à un pronostic déduit du statut de méthylation du cfDNA sérique.

### Références

Ahn S.Y. Singh V., and Tayade C, 2016. Biomarkers in endometriosis: challenges and opportunities. Fertil.Steril. 523-532.
Ahn Soo Hyun, Vinay Singh, and Chandrakant Tayade, 2017. Biomarkers in endometriosis: challenges and opportunities. Fertil.Steril. vol107, n°3, 523-532.
Beckman E.N., Léonard G.L., Pintado S.O. et al., 1985. Endometriosis of the prostate. The American Journal of Surgical Pathology, vol. 9, no. 5, pp. 374-379.
Blumenkrantz MJ, Gallagher N, Bashore RA, Tenckhoff H, 1981. Retrograde menstruation in women undergoing chronic peritoneal dialysis. Obstet Gynecol; 57:667-70
Cho S, Mutlu L, Grechukhina O, Taylor HS, 2015. Circulating microRNAs as potential biomarkers for endometriosis. Fertil Steril; 103:1252-60.e1.
Fukunaga M, 2012. Paratesticular endometriosis in a man with a prolonged hormonal therapy for prostatic carcinoma. Pathology - Research and Practice, vol. 208, no. 1, pp. 59-61.
Halme J, Hammond MG, Hulka JF, Raj SG, Talbert LM, 1984. Retrograde menstruation in healthy women and in patients with endometriosis. Obstet Gynecol; 64:151-4.
Hazout A, Montjean D, Cassuto NG, Belloc S, Dalleac A, Tesarik J and Benkhalifa M, 2018. Free Circulating Nucleic Acids and Infertility. JGWH.
Laux-Biehlmann A, d'Hooghe T, Zollner TM, 2015. Menstruation pulls the trigger for inflammation and pain in endometriosis. Trends Pharmacol Sci;36:270-6.
Liu K, Zhang W, Liu S, Dong B, Liu Y, 2015. Hepatic endometriosis: a rare case and review of the literature. Eur J Med Res; 20:48.
Mandel P, Metais P, 1948. Les acides nucléiques du plasma sanguin chez l'homme. CR Seance Soc Biol Ses Fil 142 :241-243.
Marsh, E. E. & Laufer, M. R., 2005. Endometriosis in premenarcheal girls who do not have an associated obstructive anomaly. Fertil. Steril. 83, 758-760.
Meola J, Hidalgo GDS, Rosa e Silva J, Costa Mendes Silva J, Paro Paz, and Rui Alberto Ferriani, 2013. Caldesmon: New Insights for Diagnosing Endometriosis. Biology of Reproduction. 88(5) :122,1-8.
Naqvi H, Ilagan Y, Krikun G, Taylor HS, 2014. Altered genome-wide methylation in endometriosis. Reprod Sci;21:1237-43.
Nasu K, Kawano Y, Tsukamoto Y, Takano M, Takai N, Li H, Furukawa Y, Abe W, Moriyama M, Narahara H, 2011. Aberrant DNA methylation status of endometriosis: epigenetics as the pathogenesis, biomarker and therapeutic target. J Obstet Gynaecol Res. Jul;37(7):683-95.
Petracco R, Grechukhina O, Popkhadze S, Massasa E, Zhou Y, Taylor HS, 2011. MicroRNA 135 regulates HOXA10 expression in endometriosis. J Clin Endocrinol Metab; 96:1925-33.
Rei C, Williams T, and M Feloney, 2018. Endometriosis in a Man as a Rare Source of Abdominal Pain: A Case Report and Review of the Literature, Case Reports in Obstetrics and Gynecology, vol. 2018, Article ID 2083121.
Sapkota Y, Steinthorsdottir V, Morris A.P., Fassbender A., Rahmioglu. R, 2017. Meta-analysis identifies five novel loci associated with endometriosis highlighting key genes involved in hormone metabolism. Nature communications.
Vassilopoulou L, Matalliotakis M, Zerviou M, atalliotaki C, Krithinakis K, Matalliotakis I, Spandidos D, Goulielmos G.N. Defining the enetic profile of endometriosis. Review 2019. Expérimental and Therapeutic Medicine.
Wang Y, Li Y, Yang Z, Liu K, Wang D, 2015. Genome-wide microarray analysis of long noncoding RNAs in eutopic secretory endometrium with endometriosis. Cell Physiol Biochem; 37:2231-45.
Wang WT, Sun YM, Huang W, He B, Zhao YN, Chen YQ, 2016. Genome-wide long noncoding RNA analysis identified circulating IncRNAs as novel noninvasive diagnostic biomarkers for gynecological disease. Sci Rep; 6:23343.
Zachariah R,Schmid S, Radpour R, Buerki N, Xiu-Cheng Fan A, Hahn S, Holzgreve W, Zhong XY, 2009. Circulating cell-free DNA as a potential biomarker for minimal and mild endometriosis. RBMonline: 407-411.

## Revendications

1. Méthode de diagnostic *in vitro* de l'endométriose, comprenant
(i) la mesure du taux d'ADN acellulaire dans un échantillon biologique d'un individu et
(ii) la comparaison du taux d'ADN acellulaire avec un seuil prédéterminé,
dans laquelle un taux d'ADN acellulaire supérieur au seuil prédéterminé indique que l'individu est susceptible d'avoir de l'endométriose, et un taux d'ADN acellulaire mesuré inférieur au seuil prédéterminé permet d'écarter le diagnostic d'endométriose évolutive.

2. Méthode selon la revendication 1, dans laquelle l'individu est une femme.

3. Méthode selon la revendication 1 ou la revendication 2, dans laquelle l'échantillon est un échantillon de sérum.

4. Méthode selon l'une quelconque des revendications 1 à 3, comprenant également, à partir de l'ADN acellulaire présent dans un échantillon de sérum de l'individu, une étape (iii) de détermination du niveau de méthylation d'au moins 15 gènes sélectionnés parmi les gènes décrits dans le tableau ci-dessous :
| **Nom** | **Identifiant GenBank** | **Nom** | **Identifiant GenBank** | **Nom** | **Identifiant GenBank** |
|---|---|---|---|---|---|
| CALD1 | NM_033140 | ROR2 | NM_004560 | MYO5C | NM_018728 |
| RRP1 | NM_003683 | MRPL3 | NM_007208 | COX6C | NM_004374 |
| FN1 | NM_212482 | FMNL2 | NM_052905 | MIR6133 | NR_106749 |
| FAM87B | NR_103536 | TMEM19 | NM_018279 | BRSK2 | NM_001256629 |
| TCEAL6 | NM_001006938 | ZNF438 | NM_182755 | MIR4277 | NR_036240 |
| RPL29P2 | NR_002778 | LINC01192 | NR_033945 | MIR4251 | NR_036215 |
| ATP11A-AS1 | NR_046661 | RCBTB1 | NM_018191 | MN1 | NM_002430 |
| DIP2C | NM_014974 | TSPAN33 | NM_178562 | MIR3666 | NR_037439 |
| SLCO2B1 | NM_007256 | NKD2 | NM_001271082 | AZIN1 | NM_148174 |
| RMI2 | NM_152308 | FGFR2 | NR_073009 | MIR4251 | NR_036215 |
| MIR3170 | NR_036129 | TPRG1 | NM_198485 | SLC37A2 | NM_198277 |
| LINC01007 | NR_103749 | MIR4644 | NR_039787 | FZD10 | NM_007197 |
| TSPAN17 | NM_012171 | FOXO4 | NM_005938 | STAU2-AS1 | NR_038406 |
| MIR4693 | NR_039842 | FSTL1 | NM_007085 | TDRD5 | NM_001199091 |
| HYOU1 | NM_006389 | CLMN | NM_024734 | USP1 | NM_003368 |
| TLR4 | NM_138554 | NT5C2 | NM_012229 | ACVR2A | NM_001616 |
| ADGRL3 | NM_015236 | NAV1 | NM_020443 | FBXO38 | NM_001271723 |
| IL6 | NM_000600 | SOD3 | NM_003102 | FASN | NM_004104 |
| VIRMA | NM_015496 | C3 | NM_000064 | MKRN9P | NR_033410 |
| MKRN1 | NR_117084 | UBE3A | NM_130839 | PCCA-AS1 | NR_047686 |
| INSIG1 | NM_198337 | MIR4655 | NR_039799 | | |
dans laquelle :
(i) une hyperméthylation des gènes sélectionnés parmi CALD1, RRP1, FN1, FAM87B, TCEAL6, RPL29P2, ATP11A-AS1, DIP2C, SLCO2B1, RMI2, MIR3170, LINC01007, TSPAN17, MIR4693, HYOU1, TLR4, ADGRL3, IL6, VIRMA, MKRN1, INSIG1, ROR2, MRPL3, FMNL2, TMEM19, ZNF438, LINC01192, RCBTB1, TSPAN33, NKD2, FGFR2, TPRG1, MIR4644, FOXO4, FSTL1 et CLMN, et/ou
(ii) une hypométhylation des gènes sélectionnés parmi NT5C2, NAV1, SOD3, C3, UBE3A, MIR4655, MYO5C, COX6C, MIR6133, BRSK2, MIR4277, MIR4251, MN1, MIR3666, AZIN1, MIR4251, SLC37A2, FZD10, STAU2-AS1, TDRD5, USP1, ACVR2A, FBXO38, FASN, MKRN9P et PCCA-AS1, constitue des signes évocateurs d'une endométriose.

5. Méthode selon la revendication 4, dans laquelle on mesure le niveau de méthylation d'au moins 5 gènes choisis dans le groupe constitué par CALD1, RRP1, FN1, FAM87B, TCEAL6, RPL29P2, ATP11A-AS1, DIP2C, SLCO2B1, RMI2, FBX038, ACVR2A, USP1, TDRD5 et STAU2-AS1.

6. Méthode selon la revendication 5, dans laquelle on mesure le niveau de méthylation d'au moins 10 gènes choisis dans le groupe constitué par CALD1, RRP1, FN1, FAM87B, TCEAL6, RPL29P2, ATP11A-AS1, DIP2C, SLCO2B1, RMI2, FBX038, ACVR2A, USP1, TDRD5 et STAU2-AS1.

7. Méthode selon la revendication 6, dans laquelle on mesure le niveau de méthylation des gènes CALD1, RRP1, FN1, FAM87B, TCEAL6, RPL29P2, ATP11A-AS1, DIP2C, SLCO2B1, RMI2, FBX038, ACVR2A, USP1, TDRD5 et STAU2-AS1.

8. Méthode selon l'une quelconque des revendications 1 à 7, dans laquelle le niveau de méthylation des gènes mesurés permet de confirmer le diagnostic d'endométriose.

9. Méthode selon l'une quelconque des revendications 1 à 8, dans laquelle le niveau de méthylation des gènes mesurés permet de caractériser le potentiel évolutif de l'endométriose, ce potentiel évolutif étant d'autant plus important qu'on mesure une hyperméthylation importante d'au moins 7 gènes sélectionnés parmi CALD1, RRP1, FN1, FAM87B, TCEAL6, RPL29P2, ATP11A-AS1, DIP2C, SLCO2B1, RMI2, MIR3170, LINC01007, TSPAN17, MIR4693, HYOU1, TLR4, ADGRL3, IL6, VIRMA, MKRN1, INSIG1, ROR2, MRPL3, FMNL2, TMEM19, ZNF438, LINC01192, RCBTB1, TSPAN33, NKD2, FGFR2, TPRG1, MIR4644, FOXO4, FSTL1 et CLMN, et/ou une hypométhylation importante d'au moins 3 gènes sélectionnés parmi NT5C2, NAV1, SOD3, C3, UBE3A, MIR4655, MYO5C, COX6C, MIR6133, BRSK2, MIR4277, MIR4251, MN1, MIR3666, AZIN1, MIR4251, SLC37A2, FZD10, STAU2-AS1, TDRD5, USP1, ACVR2A, FBXO38, FASN, MKRN9P et PCCA-AS1.

10. Méthode selon l'une quelconque des revendications 1 à 9, dans laquelle une hyperméthylation importante des gènes CALD1, RRP1, FN1, FAM87B, TCEAL6, RPL29P2, ATP11A-AS1, DIP2C, SLCO2B1 et RMI2 et une hypométhylation importante des gènes FBX038, ACVR2A, USP1, TDRD5 et STAU2-AS1 conduisent au diagnostic d'une endométriose susceptible de s'aggraver.

11. Kit de diagnostic pour déterminer le potentiel évolutif d'une endométriose, comprenant des réactifs pour mesurer le niveau de méthylation d'au moins 15 gènes sélectionnés parmi ceux cités à la revendication 4.

12. Kit selon la revendication 11, comprenant en outre des réactifs pour mesurer le niveau d'ADN acellulaire dans un échantillon biologique.

13. Kit de diagnostic selon la revendication 11 ou la revendication 12, comprenant des amorces et/ou des sondes spécifiques d'au moins 15 gènes tels que définis dans l'une quelconque des revendications 4 à 7.

14. Kit de diagnostic selon l'une quelconque des revendications 11 à 13, comprenant une puce oligonucléotidique sensible à la méthylation comportant des oligocléotides spécifiques d'au moins 15 gènes tels que définis dans l'une quelconque des revendications 4 à 7.

15. Kit de diagnostic selon l'une quelconque des revendications 11 à 14, comprenant des anticorps spécifiques des cytosines méthylées.


SEQUENCE LISTING

<110>  Advanced Technologies Laboratory
       Hazout, André

<120>  Utilisation du niveau d'ADN libre comme marqueur de
       l'endométriose

<130>  B190179EPA

<160>  2

<170>  PatentIn version 3.5

<210>  1
<211>  19
<212>  DNA
<213>  Artificial Sequence

<220>
<223>  amorce

<400>  1
agatttggac ctgcgagcg                                                    19


<210>  2
<211>  18
<212>  DNA
<213>  Artificial Sequence

<220>
<223>  amorce

<400>  2
gaagccgggg caactcac                                                     18